# EUROPEAN PATENT SPECIFICATION

(11) **EP 0 342 188 B1**
(45) Date of publication and mention of the grant of the patent: **21.07.1993**
(21) Application number: 87906018.4
(22) Date of filing: 01.09.1987
(51) Int. Cl.: C12N 5/00, C12N 15/00, A61K 39/395, G01N 33/577

(54) **METHOD FOR THE REMOVAL OF UNDESIRED CELLS FROM HUMAN LYMPHOCYTE POPULATIONS, APPLICATION OF THE METHOD IN MONOCLONAL ANTIBODY PRODUCTION AND KIT THEREFOR**
VERFAHREN ZUR ENTFERNUNG UNERWÜNSCHTER ZELLEN AUS MENSCHLICHEN LYMPHOZYTENPOPULATIONEN, ANWENDUNG DES VERFAHRENS ZUR HERSTELLUNG MONOKLONALER ANTIKÖRPER UND DAFÜR GEEIGNETER KIT
PROCEDE D'ELIMINATION DE CELLULES NON DESIREES DANS DES POPULATIONS DE LYMPHOCYTES HUMAINS, APPLICATION DUDIT PROCEDE DANS LA PRODUCTION D'ANTICORPS MONOCLONAUX ET KITS DESTINES A CET EFFET

(30) Priority: 04.09.1986 SE 8603711; 11.02.1987 SE 8603711
(43) Date of publication of application: 23.11.1989
(73) Proprietor: BIOINVENT INTERNATIONAL AB, S-223 70 Lund (SE)
(72) Inventor: BORREBAECK, Carl, S-222 48 Lund (SE); DANIELSSON, Lena, S-223 64 Lund (SE); MÖLLER, Susanna, S-223 68 Lund (SE)
(74) Representative: Rostovanyi, Peter
(86) International application number: SE8700389
(87) International publication number: WO8801642

(56) References cited:
- EP-A- 0 247 613
- Journal of Immunology (1985), vol. 134 no.2, pp. 786-793
- Proc. Natl. Acad. Sci. USA (1985), vol. 82, pp. 2468-2472
- Chemical Abstracts, vol. 100, 84116m, 1984, "Immunology", 51(2), 319-26
- Medline, NLM accession no.84034197, J. Immunol 1983, 131(5), 2282-90
- Chemical Abstracts, vol. 105, 72232m, 1986, Cancer Res., 46(7), 3295-8
- Chemical Abstracts, vol.96, 117049w, 1982, Dev. Biol. 90(1), 91-8
- Tibtech, June 1986, vol. 4, p. 147-53, "In vitro immunization for production of murine and human monoclonal antibodies : present status", C A K Borrebaeck

## Description

The present invention comprises a method for the removal of, for in vitro immunisation, undesired cells from cell populations containing human lymphocytes, a method in the in vitro immunisation of lymphocytes for use in the production of human monoclonal antibodies, and a kit for in vitro immunisation of lymphocytes for use in the production of human monoclonal antibodies.

### Background of the invention

Monoclonal antibodies were introduced in 1975 by Köhler and Milstein. The concept implies fusing immune lymphocytes with a continuous cell line, for example myeloma. A cloning and selection method makes it possible to select and culture cells producing a specific antibody. This cell clone then originates from one original cell ("monoclonal") and produces exactly identical copies of a specific antibody. These monoclonal antibodies have been prepared against a long line of molecules, and this type of antibody has been used and is still being used to an immense extent. Also the commercial development has been considerable, and today a large number of monoclonal antibodies are being marketed, above all for diagnostic purposes.

This development of mouse-monoclonal antibodies (i.e. prepared by means of immune mouse lymphocytes) has not been followed up with human monoclonal antibodies, in spite of the extensive prognosticised therapeutic use of these antibodies. This is due to the fact that it is extremely difficult to produce, in a practically feasible manner, immune human lymphocytes for immortalisation by cell hybridisation or transfection. Ethically, patients, volunteers etc. cannot today be immunised with the molecules against which it is desired to produce human monoclonal antibodies, i.e. tumour-associated antigens, bacterial and viral antigens, toxins etc. Up to the present, the procedure was to seek out patients suffering from, for example, infections, tumours etc., thereby to gain access to immune lymphocytes (so-called in vivo sensitised lymphocytes). From the practical point of view, this technique is unacceptable.

The development therefore has occurred within so-called in vitro immunisation, implying that non-immune human lymphocytes have been immunised in cell culture environment, thereby to ensure that all types of antigens can be used. These in vitro immunised human lymphocytes are then fused with myeloma or lymphoblastoid cells for continuous production of human monoclonal antibodies. Alternatively, the immune lymphocytes can be transfected with a suitable viral or bacterial genome, thereby to immortalise the cells.

In other words, in vitro immunisation is the only technique by which human monoclonal antibodies can be prepared in future in a practically feasible and ethically acceptable manner. The technique has been developed during a number of years in the murine system, and there exist today, for mouse cells, in vitro immunisation methods which function well (Borrebaeck, 1986).

In the human system to which considerable resources have been made available in order to provide for in vitro immunisation, there is today nothing which corresponds to the mouse system. This is because the activation requirements for human cells are partly different, and above all because peripheral blood lymphocytes have been used which, possibly, exist in a "deeper" state of rest. Still there are some reports on human in vitro immunisation against a few types of haptens, for example bombesin (Ho et al. 1985) and DNP-HSA (Teng et al. 1985), and red corpuscles (Strike et al. 1984; Hoffman & Hirst 1985). These in vitro immunisation systems have no lowest common denominator, and different techniques have been used with very different results, frequently with very low yields of specific hybridomes. In addition, there is at present no system at all capable of producing a satisfactory primary immunological response in vitro against thymus dependent antigens. Using different types of T-cell derived lymphokines is one technique which has functioned well in connection with mouse cells (Borrebaeck & Möller 1986) but which alone is not sufficient in the human system. Other attack techniques that have been tried in order to support a human in vitro immunisation for the production of human monoclonal antibodies comprise using
(1) adjuvant peptides, such as muramyl dipeptide;
(2) monokine supplemented medium;
(3) separation of cell populations with Sepharose/Sephadex, gelatin, plastics nylon wool adherence, antibody spanning, complement lysis, affinity column;
(4) polyclonal activators, such as endotoxins (LPS), lectins (PHA, PWM, Con A), Staphylococcus aureus cells, protein A or G;
(5) special sera, such as ABO, FCS, rabbit serum.

It has now been found that the problem primarily lies int. al. in the removal of cell subpopulations (possibly cytotoxic) capable of suppressing or otherwise preventing the antigen-specific immunological response in vitro.

### Object and characteristics of the invention

It therefore is an object of the present invention to provide a method for the removal of, for the in vitro immunisation, undesired cells from human lymphocyte containing cell populations, thereby to permit production of a sufficient number of antigen-specifically in vitro activated human lymphocytes which are then further used as a fusion or transfection partner in the production of human monoclonal antibodies. This method of removing undesired subpopulations of cells from human blood, tonsils, lymphatic nodes, spleen cells, bone marrow etc., which up to the present have prohibited a high yield of specifically in vitro immunised human lymphocytes, is characterised by using so-called lysosomotropic agents. These agents possess the specific ability to kill all lysosome containing cells, such as monocytes/macrophages, NK cells and possibly other and at present unknown cell subpopulations in as short a time as 30-40 min (Thiele et al, 1983, 1986).

This method thus affords the possibility of adapting in some thirty or forty minutes, and without extensive cell separation experiments (where presently no satisfactory methods are available and, what is more, one does not know exactly which cell one is looking for), the lymphocyte population so that it can be used for in vitro immunisation experiments for the production of human monoclonal antibodies. This is possible because the B cells of the lymphocyte population have now been given the possibility of being activated antigen-specifically without any negative effects from lysosomal-positive cells. In this manner, an immune lymphocyte population has been produced which can be used as the only presently available production source of human monoclonal antibodies. This again means that it is possible to start producing human monoclonal antibodies to the same extent as mouse monoclonal antibodies. The immense in vivo potential of human monoclonal antibodies in the treatment of tumours, in locating tumours, poisonings, in preventing transplant rejections etc., can then be exploited.

A further object of the invention is to provide a method in the vitro immunisation of lymphocytes for use in the production of human monoclonal antibodies, in which method lysosomotropic agents, derivatives thereof, or substances synthetised on the basis of these agents are caused to act in vitro on human lymphocyte containing cell populations for the removal of cell populations having a negative effect on the in vitro immunisation, whereupon the lymphocytes are in vitro immunised antigen-specifically and immortalised. The immortalisation may be effected by fusion with murine or human myelomas, lymphoblastoid cell lines, lymphoma cells, or Epstein-Barr viral infection. Furthermore, the immortalisation may be effected by transfecting the immune antigen-specific cells with viral, bacterial or mammal genomes.

The lysosomotropic agents that can be utilised for the method are amino acid esters (Goldman & Kaplan 1973) of the type L-leucine methyl ester (Leu-OMe), L-glutamic acid dimethyl ester etc. Also derivatives of lysosomotropic amino acids or peptides based on these derivatives can be used. These agents penetrate the plasma cell membrane and diffuse freely into the lysosomes where they are quickly metabolised to free amino acids. These free and more polar amino acids are unable to diffuse out of the lysosomes as quickly as the methyl esters diffuse in, which entails a rapid increase in the intralysosomal osmotic pressure and a subsequent rupture of these organelles. However, we do not wish to link up the behaviour of the lysosomotropic substances with any specific theory.

The invention also comprises a kit for the removal of, for in vitro immunisation, undesired cells from human lymphocyte containing cell populations. The so treated cell populations may then be used for the production of monoclonal antibodies. The kit includes a container which contains lymphokines and a further container which contains lysosomotropic agents, derivatives thereof, or substances synthetised on the basis of these agents. In addition, the kit preferably contains some type of immune response modifying agent, so-called BRM (Biological Response Modifier) and different disposable materials adapted to promote the effect of the reagents on the cell populations, as well as directions for use.

The invention will be described in more detail in the following Examples.

### Example 1

Human peripheral lymphocytes were purified by means of density centrifugation on a Ficoll gradient. The lymphocytes (10 x 10⁶ cells/ml) were treated with 0.45 mg L-leucine methyl ester/ml for 40 min. at 25^{o}C. The medium shall have a low serum level, for which reason RO (RPMI 1640 with 0% fetal calf serum (FCS)) was used. The cells were then washed 2 times in R2-5 (RPMI 1640 with 2-5% FCS), whereupon they were added to the in vitro immunisation for production of human monoclonal antibodies. The duration of the in vitro immunisation was 6 days, and the immunogen used was 1 µg KLH/ml. The culture also contained the lymphokines TRF, BCDF, IL-1/2 On day 6, it was tested how many antigen-specific plaque-forming cells had been formed. This number gives a measure of how well the in vitro immunisation functions. Cells not treated with Leu-OMe were used for control. The result is shown in Table 1.

**Table 1**

| Cells | Number of plaque-forming cells/10⁶ B cells | |
|---|---|---|
| | KLH | Gelatin* |
| Leu-OMe-treated cells** | 1156 | 19 |
| Untreated cells | 7 | 11 |

| | | |
|---|---|---|
| * Gelatin was used as control antigen in the plaque test. | | |
| ** In this Example, human peripheral blood lymphocytes were used. | | |

### Example 2

Human peripheral lymphocytes (PBL) which had been in vitro immunised according to Example 1 with 1 µg KLH/ml for 6 days, were immortalised by fusion with Sp2/0-Agl4 myeloma cells or WIL2-UC729HF2 lymphoblastoid cells by means of polyethylene glycol. After 14-21 days the antigen-specific antibody production of the growing hybrids was tested. The result is shown in Table 2.

**Table 2**

| Cells* | number of hybrids**/number of tested cells | specific efficiency (%) |
|---|---|---|
| Untreated PBL | 0/96 | 0 |
| Leu-OMe-treated PBL | 17/96 | 18 |

| | | |
|---|---|---|
| * Human lymphocytes fused with Sp2/0-Agl4. The result with WIL2-UC729HF2 was the same, with absolute figures that were lower by about 30%. | | |
| ** Number of hybrids which were shown by the test to produce antigen-specific antibodies. | | |

### Example 3

Human peripheral blood lymphocytes (PBL) were isolated from healthy donors by density centrifugation and further separated into B, T and accessory (A) cells, as described recently (Danielsson, L., Möller, S.A. & Borrebaeck, C.A.K. Immunology 61, 51-55 (1987)). PBL were fractionated into T and non-T cells by rosetting with 2-amino ethyl(isothiouronium bromide)treated sheep red corpuscles, and the latter cell population was incubated on Petri dishes coated with fibronectin or autologous plasma. Non-adherent cells (B cells) were decanted, and adherent cells (accessory cells) were removed by 10 mM EDTA. The B cells were stimulated with 50 µg Staphylococcus aureus Cowan I/ml and irradiated (2000 R) T cells with 10 µg PWM/ml overnight. The accessory cells were stimulated with 5 IU gamma interferon/ml and 10 µm indomethacin. The cell populations were cultured in supplemented RPMI 1640 which contained 10% human AB serum at a cell ratio of 2:1:0.4 (T:B:A) for a total of 6 days. The antigenic dose was 1 µg/ml. The culture was supplemented with recombinant IL-2 (5 U/ml) and sPWM-T (25% by vol.), produced as described (Danielsson, L., Möller, S.A. & Borrebaeck C.A.K. Immunology 61, 51-55 (1987)). T cells (10⁷ cells/ml) suspended in serum-free RPMI 1640 were incubated with 2.5 mM freshly prepared Leu-OMe for 40 min. at room temperature. The cells were then washed 3 times in RPMI 1640 which contained 2% human AB serum.

The in vitro immunised cells were tested against the immunogen KLH, an unrelated antigen (gelatin) and against anti-Ig (to give the total number of Ig-secreting cells), using a filter immuno-plaque assay (Danielsson, L., Möller, S.A. & Borrebaeck C.A.K. Immunology 61, 51-55 (1987) and Möller, S.A. & Borrebaeck, C.A.K. J. Immunol. Methods 79, 195-204 (1985)). The plaque number is the mean value of three assays. The results are shown in Table 3.

**Table 3**

| Effect of O-methyl-leucine on in vitro immunisation of human peripheral B lymphocytes | | | | | | | | | |
|---|---|---|---|---|---|---|---|---|---|
| | KLH | Leu-Ome | sPWM-T | IL-2 | A-cell | T-cell | number of PFC*/10⁶ B cells | | |
| | | | | | | | KLH | gelating | anti-Ig |
| 1. | - | - | - | - | + | + | 6 | 0 | 1190 |
| 2. | + | - | - | - | + | + | 5 | 0 | 1400 |
| 3. | + | - | + | + | + | + | 112 | 3 | 9500 |
| 4. | - | - | + | + | + | + | 14 | 0 | 7950 |
| 5. | + | + | + | + | + | + | 1021 | 0 | 15360 |
| 6. | - | + | + | + | + | + | 25 | 4 | 13500 |

| | | | | | | | | | |
|---|---|---|---|---|---|---|---|---|---|
| * The plaque assay was conducted as described in Example 3. The plaque number is the mean value of there assays. | | | | | | | | | |

Thus, if the Leu-OMe sensitive T cell population was removed, and if the remaining T cells were tested together with isolated B and A cells in the same in vitro immunisation system, the number of plaque-forming cells increased by a factor 10.

### Example 4

PBL were isolated from healthy donors, using density centrifugation, and treated with 2.5 mM Leu-OMe, as described in Example 3. The mean number of cells recovered after the Leu-OMe treatment was 70% (n = 22). Leu-OMe-treated PBL were suspended in RPMI 1640 supplemented with 1% by vol. nonessential amino acids, 5 mM L-glutamine, streptomycin (50 µg/ml), penicillin (50 UI/ml), 50 µM 2-mercaptoethanol, and 10% human AB0 serum. The serum was collected from healthy blood donors. Cytokines (IL-2, sPWM-T, gamma-interferon, IL-1, BCDF) and 1 µg KLH/ml were then added to the culture. The final cell concentration was 3.5 x 10⁶ cells/ml and 4 ml (6 well plate) or 30 ml (75 m² flask) cultures were used. The cells were cultured for 6 days and re-fed on day 3-4 with further medium (20% of original culture volume). sPWM-T contained gamma-interferon (400 U/ml), IL-2 (20 U/ml) and B cell growth and differentiation activities. No significant variation was observed between different batches of sPWM-T when tested in vitro immunisations. The results are shown in the Table below.

**Table 4**

| Effect of O-methyl-leucine, IL-2 and gamma-interferon on in vitro immunisation of unseparated human peripheral lymphocytes | | | | | | | | |
|---|---|---|---|---|---|---|---|---|
| | KLH | Leu-Ome | IL-2 | IFN | sPWM-T | number of PFC*/10⁶ B cells** | | |
| | | | | | | KLH | gelatin | anti-Ig |
| 1. | + | - | - | - | - | 0 | 0 | 175 |
| 2. | + | - | 5# | 2.5# | + | 12 | 3 | 1530 |
| 3. | - | + | 5 | - | + | 54 | 8 | 26540 |
| 4. | + | + | - | - | + | 1225 | 38 | 33450 |
| 5. | + | + | 1 | - | + | 1150 | 8 | 36780 |
| 6. | + | + | 2.5 | - | + | 1415 | 12 | 34900 |
| 7. | + | + | 5 | - | + | 1445 | 12 | 34000 |
| 8. | + | + | 10 | - | + | 1190 | 0 | 32450 |
| 9. | + | + | 50 | - | + | 945 | 0 | 36800 |
| 10. | + | + | 500 | - | + | 780 | 12 | 18830 |
| 11. | + | + | 5 | 100 | + | 1400 | 16 | 28000 |
| 12. | + | + | 5 | 500 | + | 1340 | 0 | 20400 |
| 13. | + | + | 5 | 2000 | + | 1370 | 0 | 21600 |

| | | | | | | | | |
|---|---|---|---|---|---|---|---|---|
| * The plaque assay was conducted as described in Example 3. The plaque number is the mean value of three assays. | | | | | | | | |
| ** The number of B cells was determined by staining to show surface-bound Ig. | | | | | | | | |
| # U/ml | | | | | | | | |

These results show that human peripheral lymphocytes can be directly in vitro immunised with comparable efficiency to separated and Leu-OMe-treated cells, whereby several complicated cell separation steps are eliminated.

### Example 5

In vitro immunised PBL (1 µg KLH/ml) and malign fusion partner were mixed at a ratio of 2:1 and fused using 30% (HF₂) or 45% (NS-l/Sp2/0) polyethylene glycol (molecular weight 1540) with 7% dimethyl sulphoxide (Borrebaeck, C.A.K. Scand. J. Immunol. 18, 9-12 (1983)). The human x human hybrids were resuspended in supplemented RPMI 1640 containing 10% fetal calf serum, 1 mM sodium pyruvate, 132 µg oxaloacetic acid/ml, 100 µM hypoxanthine, 0.4 µM aminopterin, 16 µM thymidine and 15% by vol. HF₂-conditioned medium. Mouse myeloma cells were fused and cloned, except that feeder cells were omitted. Both human x human and human x mouse hybrids were plated out in 24 well plates (0.7 -1.0 x 10⁶ cells/well). The hybridomas were screened for production of specific antibodies, using an enzyme immunoassay. To sum up, 96 microtiter wells were each coated with 0.3 µg KLH by allowing the antigen solution to dry in the well. Gelatin (0.1%) was then used in order to block the wells for 30 min. at 37^{o}C. The hybridoma supernatant (100 µl/well) was added to the washed wells and allowed to react for 30 min. at 37^{o}C. Peroxidase-conjugated anti-human Ig antibodies diluted in phosphate-buffered saline solution containing 10% fetal calf serum (100 µl/well) were finally incubated for 60 min., and enzyme substrate (ABTS) was added to develop the immunoassay. The results are shown in the Table below.

**Table 5**

| Human x human and human x mouse hybridomas produced by using human peripheral lymphocytes in vitro immunised with KLH | | | | |
|---|---|---|---|---|
| | Fusion partner | Number of viable hybrids* | Number of specific hybrids* | Specific efficiency (%) |
| 1. | HF₂ | 94/96 | 3/94 | 3.2 |
| 2. | HF₂ | 96/96 | 5/96 | 5.2 |
| 3. | HF₂ | 96/96 | 7/96 | 7.3 |
| 4. | HF₂ | 96/96 | 12/96 | 12.5 |
| 5. | HF₂ | 96/96 | 14/96 | 14.6 |
| 6. | Sp2/0 | 96/96 | 0/96 | 0 |
| 7. | Sp2/0 | 96/96 | 4/96 | 4.2 |
| 8. | NS-l | 96/96 | 3/96 | 3.1 |
| 9. | NS-l | 72/72 | 4/72 | 5.5 |

| | | | | |
|---|---|---|---|---|
| * Number of growth positive wells/total number of seeded wells # Number of antibody-positive wells/number of growth-positive wells. Antibody-positive value = >4 x the background value (OD 0.120-0.150), as determined by enzyme immunoassay. | | | | |

Human x human hybridomas secreting antibodies specific to hemocyanin could easily be detected, as will appear from the above results. The specific efficiency (number of wells producing specific antibodies in relation to the number of wells exhibiting cell growth x 100) was in the range 3-15%. Human x mouse hybridomas gave a somewhat lower specific efficiency, although the positive values were more consistent (3-5%).

The anti-hemocyanin antibodies were also tested in the enzyme immunoassay against uncoated microtiter wells or wells coated with 0.5 µg bovine serum albumin/well or 0.1% gelatin to ensure that the reactivity was not due to nonspecific binding (Haskard, D.O., Gul, V. & Archer, J.R. J. Immunol. Methods 77, 291-295 (1985)).

### Example 6

The fusion of in vitro immunised cells and the enzyme immunoassay was performed as described in the legends of Table 5 (Example 5) and Table 6 below. The gelatin blocking step was, however, omitted in the enzyme immunoassay. Digoxin was conjugated to transferrin (Butler, V.P. & Chen. J.P. Proc. Natl. Acad. Sci. (U.S.A) 57, 71-78 (1967) and 1 µg digoxin-transferrin/ml was used for in vitro immunization. The molar ratio of digoxin to transferrin was approximately 5:1. Digoxin conjugated to bovine serum albumin (0.5 µg/well) was used in the enzyme immunoassay. The results are shown in the Table below.

**Table 6**

| Human x human and human x mouse hybridomas produced by using human peripheral lymphocytes in vitro immunized with digoxin | | | | |
|---|---|---|---|---|
| | Fusion partner | Number of viable hybrids* | Number of specific hybrids* | Specific efficiency (%) |
| 1. | HF₂ | 118/120 | 2/118 | 1.7 |
| 2. | HF₂ | 144/144 | 5/144 | 3.5 |
| 3. | NS-l | 144/144 | 13/144 | 9.0 |
| 4. | NS-l | 144/144 | 12/144 | 8.3 |
| 5. | NS-l | 144/144 | 13/144 | 9.0 |
| 6. | NS-l | 144/144 | 8/144 | 5.5 |

| | | | | |
|---|---|---|---|---|
| * Number of growth-positive wells/total number of wells seeded. # Number of antibody-positive wells/number of growth-positive wells. Antibody-positive value = >3-4x (human x human hybridomas) or >4x (human x mouse hybridomas) the background value (OD 0.100-0.110). | | | | |

As will be evident from the results, human x human hybridomas specific to the immunogenic hapten could be produced, although the specific efficiency was somewhat lower compared to when hemocyanin was used as immunogen. However, no anti-digoxin antibodies were detected if the immunogen was omitted from the culture. Also human x mouse heterohybridomas specific to digoxin were produced, using NS-l as the malign fusion partner. The specific efficiency was high, and several hybrids produced antibodies which proved to be strongly positive in the enzyme immunoassay (> 7-8 x background-OD value). The specificity was tested as described for anti-hemocyanin antibodies (Haskard, D.O., Gul, V. & Archer, J.R. J. Immunol. Methods 77, 291-295 (1985)). The anti-digoxin specific hybridomas were then cloned three times by limiting dilution during a culture period of 10 weeks. After this time, approximately 35% of the original hybridomas still produced antibodies specific to digoxin.

### References:

Borrebaeck, C.A.K. (1986) TIBTECH. 4, 147
Borrebaeck, C.A.K. & Möller, S.A. (1986) J. Immunol. 136, 3710
Danielsson, L., Möller, S.A. & Borrebaeck, C.A.K. (1986) Immunology 61, 51
Goldman, R. & Kaplan, A. (1973) Biochim. Biophys. Acta 318, 205
Ho, M.K., Rand, N. Murray, J., Kato, K. & Rabin, H. (1985) J. Immunol. 135, 3831
Hoffman, M.K. & Hirst, J.A. (1985) in HUMAN HYBRIDOMAS AND MONOCLONAL ANTIBODIES (Engleman, Foung, Larrick & Raubitschek, Eds.), pp. 277-289, Plenum Press
Strike, L.E., Devens, B.H. & Lundak, R.L. (1984) J. Immunol. 132, 1798
Teng, N.N.H., Reyes, G.R., Bieber, M., Fry, K.E., Lam, K.S. & Hebert, J.M. (1985) in HUMAN HYBRODOMAS AND MONOCLONAL ANTIBODIES (Engleman et al. Eds.), pp. 71-91, Plenum Press.
Thiele, D.L., Kurosaka, M. & Lipsky, P.E. (1983) J. Immunol. 131, 2282
Thiele, D.L. & Lipsky, P.E. (1986) J. Immunol. 136, 1038

## Claims

1. A method of generation of human monoclonal antibodies comprising the steps of in vitro removal of undesired cells from human lymphocyte containing cell populations, antigen specific immunization and immortalization, **characterized** in that lysosomotropic agents or derivates thereof are caused to act in vitro on human lymphocyte containing cell populations for the removal of cell subpopulations having a negative effect on the generation of antigen-specific human monoclonal antibodies.

2. A method as claimed in claim 1, **characterized** in that the lysosomotropic agent employed consists of lysosomotropic amino acid derivatives or peptides based on these derivatives.

3. A method as claimed in claim 1, **characterized** in that the lysosomotropic agent employed is L-leucine-O-methyl ester, or peptides based on L-leucine--O-methyl ester.

4. A method as claimed in one or more of claims 1-3, **characterized** in that the immune antigen specific lymphocytes are immortalized by fusion with murine or human myelomas, lymphoblastoid cells, lymphoma cells or Epstein-Barr viral infection.

5. A method as claimed in one or more of the claims 1-3, **characterized** in that the immune antigen specific lymphocytes are immortalized by transfection with viral, bacterial or mammal genomes.

## Patentansprüche

1. Verfahren zur Erzeugung menschlicher monoklonaler Antikörper, umfassend die Schritte der in vitro-Entfernung unerwünschter Zellen aus menschliche Lymphozyten enthaltenden Zellpopulationen, der antigenspezifischen Immunisierung und der Immortalisierung, **dadurch gekennzeichnet, daß** lysosomotrope Agenzien oder ihre Derivate veranlaßt werden, in vitro, zur Entfernung von Zellsubpopulationen, die einen negativen Einfluß auf die Erzeugung antigenspezifischer, menschlicher monoklonaler Antikörper aufweisen, auf die menschliche Lymphocyten enthaltenden Zellpopulationen einzuwirken.

2. Verfahren nach Anspruch 1, **dadurch gekennzeichnet, daß** das verwendete lysosomotrope Agens aus lysosomotropen Aminosäurederivaten oder aus auf diesen Derivaten beruhenden Peptiden besteht.

3. Verfahren nach Anspruch 1, **dadurch gekennzeichnet, daß** das verwendete lysosomotrope Agens L-Leucin-O-methylester ist oder aus auf L-Leucin-O-methylester basierenden Peptiden besteht.

4. Verfahren nach einem oder mehreren der Ansprüche 1 bis 3, **dadurch gekennzeichnet, daß** die immunantigenspezifischen Lymphozyten durch eine verschmelzung mit menschlichen oder Nagetier-Myelomzellen, lymphoblastoiden Zellen, Lymphomzellen oder durch eine Epstein-Barr-Virusinfektion immortalisiert werden.

5. Verfahren nach einem oder mehreren der Ansprüche 1 bis 3, **dadurch gekennzeichnet, daß** die immunantigenspezifischen Lymphozyten durch Transfektion mit viralen, bakteriellen oder Säugetier-Genomen immortalisiert werden.

## Revendications

1. Méthode de production d'anticorps monoclonaux humains comprenant les étapes d'élimination in vitro de cellules non désirées dans des populations de cellules contenant des lymphocytes humains, d'immunisation spécifique vis-à-vis d'antigènes et d'immortalisation, caractérisée en ce que l'on fait agir des agents lysosomotropes ou des dérivés de ces agents in vitro sur des populations de cellules contenant des lymphocpes humains pour éliminer les sous-populations de cellules ayant un effet négatif sur la production d'anticorps monoclonaux humains spécifiques d'antigènes.

2. Méthode comme revendiquée dans la revendication 1, caractérisée en ce que l'agent lysosomotrope utilisé est constitué par des dérivés d'amino acides lysosomotropes ou des peptides à base de ces dérivés.

3. Méthode comme revendiquée dans la revendication 1, caractérisée en ce que l'agent lysosomotrope utilisé est l'ester de méthyle de la L-leucine ou des peptides à base d'ester de méthyle de la L-leucine.

4. Méthode comme revendiquée dans une ou plusieurs des revendications 1-3, caractérisée en ce que les lymphocytes immuns spécifiques d'antigènes sont immortalisés par fusion avec des cellules lymphoblastoïdes, des cellules de lymphomes, une infection de virus d'Epstein-Barr ou des myélomes murins ou humains.

5. Méthode comme revendiquée dans une ou plusieurs des revendications 1-3, caractérisée en ce que les lymphocytes immuns spécifiques d'antigènes sont immortalisés par transfection avec des génomes de virus, de bactéries ou de mammifères.
